# FASCICULE DE BREVET EUROPEEN

(11) **EP 1 644 312 B1**
(45) Date de publication et mention de la délivrance du brevet: **19.05.2010**
(21) Numéro de dépôt: 04767629.1
(22) Date de dépôt: 08.07.2004
(51) Int. Cl.: C07C 67/31, C07C 51/09, C07C 319/14, C07C 323/52, C07C 69/712, C07C 59/88

(54) **PREPARATION DE DERIVES DE 1,3-DIPHENYLPROP-2-EN-1-ONE**
HERSTELLUNG VON 1,3-DIPHENYLPROP-2-EN-1-ONDERIVATEN
PREPARATION OF 1,3-DIPHENYLPROP-2-EN-1-ONE DERIVATIVES

(30) Priorité: 08.07.2003 FR 0308354
(43) Date de publication de la demande: 12.04.2006
(73) Titulaire: Genfit, 59120 Loos (FR)
(72) Inventeur: DELHOMEL, Jean-François, F-62144 ACQ (FR); CAUMONT-BERTRAND, Karine, F-59236 Frelinghien (FR)
(74) Mandataire: Tezier Herman, Béatrice
(86) Numéro de dépôt international: PCT/FR2004/001797
(87) Numéro de publication internationale: WO 2005/005369

(56) Documents cités:
- GB-A- 1 469 845
- US-A- 4 656 305
- SZAJDA M ET AL: "New alkoxycarbonylalkyloxychalcones and their alpha, beta-dibromo derivatives of potential antimicrobial activity" PHARMAZIE, VEB VERLAG VOLK UND GESUNDHEIT. BERLIN, DD, vol. 44, no. 3, mars 1989 (1989-03), pages 190-191, XP002271328 ISSN: 0031-7144
- DATABASE CAPLUS 12 mai 1984 (1984-05-12), XP002271330 Database accession no. 1968:426935 & PALANOWSKI RYSZARD ET AL: "SYNTHESIS OF POTENTIAL VASOACTIVE COMPOUNDS. I. PHENYLACRYLOPHENONE DERIVATIVES" ACTA POLONIAE PHARMACEUTICA, vol. 24, no. 6, 1967, pages 567-574,
- DATABASE CAPLUS 12 mai 1984 (1984-05-12), XP002271332 Database accession no. 1984:51185 & SAFAK O CIHAT ET AL: "CHALCONES. II. SYNTHESIS OF SOME CHALCONE DERIVATIVES AND THEIR ANTIFUNGAL ACTIVITY AGAINST CANDIDA ALBICANS" FABAD FARMASOTIK BILIMLER DERGISI, vol. 8, no. 2, 1983, pages 80-88,
- DATABASE CAPLUS 19 février 1994 (1994-02-19), XP002271333 Database accession no. 1994:77943 & JP 05 255655 A (KANEBO) 5 octobre 1993 (1993-10-05)
- PATENT ABSTRACTS OF JAPAN vol. 0141, no. 26 (C-0699), 9 mars 1990 (1990-03-09) & JP 02 003670 A (NIPPON OIL & FATS CO LTD), 9 janvier 1990 (1990-01-09)

## Description

La présente invention concerne un nouveau procédé de préparation de dérivés de 1,3-diphénylprop-2-èn-1-one substitués sur un des deux groupes phényle, par un groupement carboxyalkyloxy ou un groupement carboxyalkylthio.

Les 1,3-diphénylpropèn-1-ones sont préparées de façon générale par réaction de condensation d'un aldéhyde avec une cétone selon la réaction de Claisen Schmidt ( March J, 1992 « Advanced Organic Chemistry », Fourth Edition, 940, Wiley Interscience).

De manière classique, les 1,3-diphénylprop-2-èn-1-ones substituées par un groupement carboxyalkyloxy sont obtenues selon cette méthode à partir des matières premières (aldéhyde et cétone) choisies de façon à ce quelles soient substituées par un groupement carboxyalkyloxy ou par l'ester correspondant. Cette succession d'étapes peut être résumée par l'un des schémas réactionnels suivants :

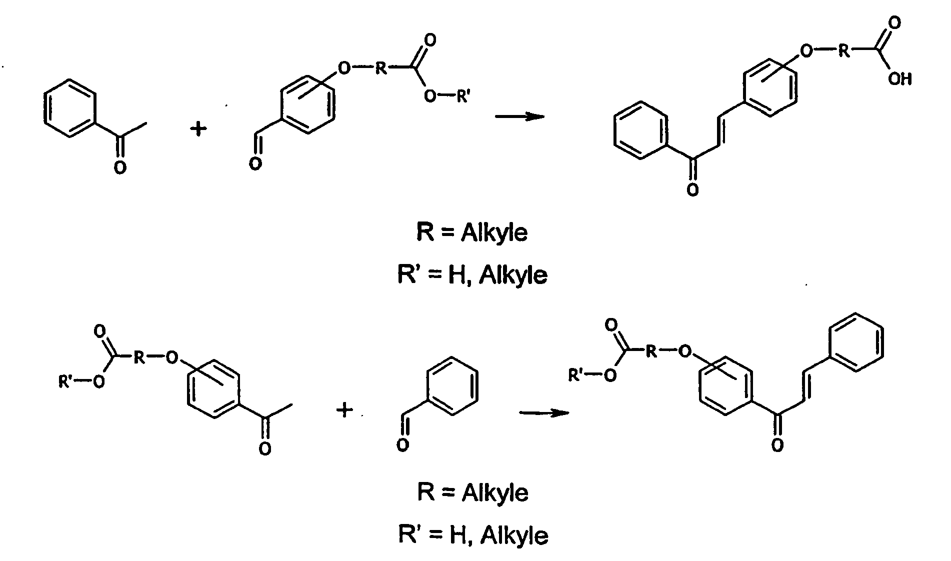

Cependant, le caractère acide du composé obtenu et la présence fréquente dans le milieu réactionnel de produits secondaires et de matières premières n'ayant pas réagi rendent la purification par recristallisation ou par chromatographie sur gel de silice délicate et occasionnent une diminution significative du rendement.

Ainsi, l'utilisation de cette stratégie de synthèse pour la préparation des composés cités en tant qu'exemple 1 et exemple 3 dans la présente demande n'a pas permis d'atteindre des rendements totaux supérieurs à 10% (US 46 56 305).

Les inventeurs ont maintenant conçu un procédé simple à mettre en oeuvre permettant d'obtenir les 1,3-diphénylpro-2-pèn-1-ones substituées par un groupement carboxyalkyloxy ou un groupement carboxyalkylthio, avec des rendements élevés. Le procédé se distingue de la synthèse précédemment décrite en ce que la fonction carboxyalkyloxy ou carboxyalkylthio est introduite sous la forme de l'ester tertiobutylique par réaction avec un dérivé de 1,3-diphénylprop-2-èn-1-one substitué par un groupement hydroxyle ou thiol. Ce dernier est généralement obtenu par la réaction de Claisen Schmidt.

L'un des moyens du procédé selon l'invention réside dans l'utilisation du groupement protecteur acido-sensible de l'acide carboxylique de type tertiobutyle. Les inventeurs ont mis en évidence que ce groupement peut être introduit et clivé dans des conditions compatibles avec la structure chimique des diphényl-1,3-prop-2-én-1-ones. Ils ont tiré parti de ces avantages pour la mise au point d'une nouvelle méthode de synthèse qui fait l'objet de l'invention.

Les 1,3-diphénylprop-2-èn-1-ones substituées par un groupement carboxyalkyloxy ou un groupement carboxyalkylthio ainsi obtenues présentent un grand intérêt dans le domaine pharmaceutique ou cosmétique. En effet, ces composés présentent à la fois des propriétés d'activateurs PPAR, d'antioxydants et d'antiinflammatoires et, à ces titres, ils présentent un haut potentiel thérapeutique ou prophylactique et en particulier sont utilisables pour traiter ou prévenir des pathologies vasculaires cérébrales, les maladies cardiovasculaires, le syndrome X, la resténose, le diabète, l'obésité, l'hypertension, les maladies inflammatoires, les cancers ou néoplasmes (tumeurs bénignes ou malignes), les maladies neurodégénératives, dermatologiques et les désordres liés au stress oxydatif, pour prévenir ou traiter les effets du vieillissement en général et par exemple le vieillissement cutané, notamment dans le domaine cosmétique (l'apparition de rides, etc.).

La présente invention a donc pour but de proposer un procédé de préparation de dérivés de 1,3-diphénylprop-2-èn-1-one substitués sur un des deux groupes phényle par un groupement carboxyalkyloxy ou carboxyalkylthio simple à mettre en oeuvre et permettant d'obtenir des rendements élevés.

Ce but et d'autres sont atteints par la présente invention qui a notamment pour objet un procédé de préparation de dérivés de 1,3-diphénylprop-2-èn-1-one substitués par un groupement carboxyalkyloxy ou carboxyalkylthio, comprenant une étape, appelée ci-dessous (i), de mise en contact d'au moins un dérivé de 1,3-diphénylprop-2-èn-1-one substitué sur un des groupes phényl par un groupement hydroxyle ou thiol avec au moins un composé halogéné de formule générale (II) présentée ci dessous, dans laquelle Y représente un atome d'halogène, R est une chaîne alkyle de C1-C24 (comportant de 1 à 24 atomes de carbone) et R' est un groupement tertiobutyle choisi comme groupement protecteur acido-sensible de l'acide carboxylique.

Cette étape peut être résumée par l'un des schémas réactionnels suivants : 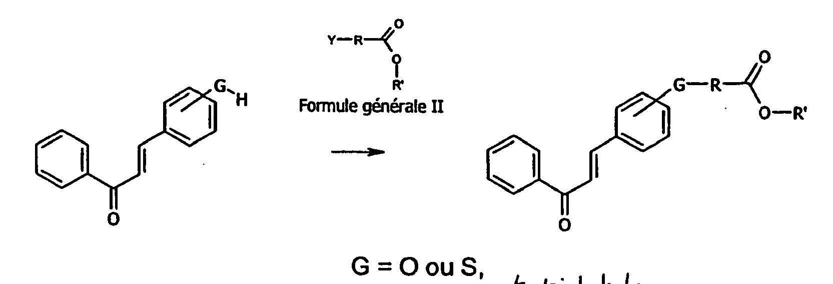
R = chaîne alkyle et R' = groupement tertiobutyle
Etape (i) : première illustration

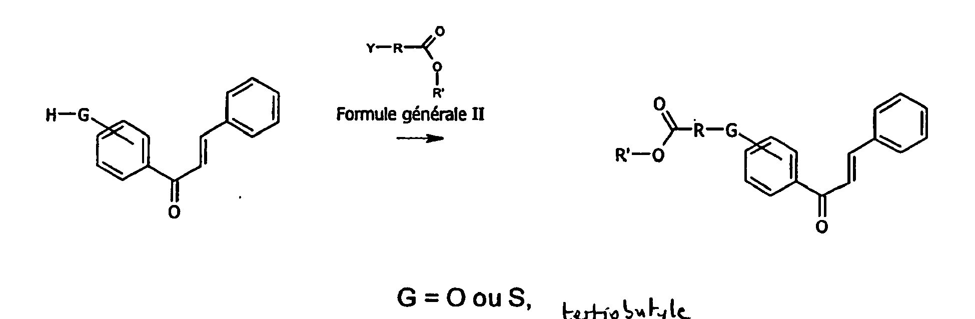

R = chaîne alkyle et R' = groupement tertiobutyle
Etape (i) : seconde illustration

Cette étape est avantageusement réalisée à une température comprise entre 25 et 120°C et plus préférentiellement entre 80 et 120°C, de préférence à pression atmosphérique, en présence d'un catalyseur, tel que le carbonate de potassium ou de césium.

De manière préférentielle, cette étape est reproduite en ajoutant plusieurs fois du composé halogéné de formule générale (II) et éventuellement du catalyseur, tel que le carbonate de potassium ou de césium, avantageusement jusqu'à disparition du dérivé de 1,3-diphénylprop-2-èn-1-one substitué sur un des groupes phényle par un groupement hydroxyle ou thiol.

La chaîne alkyle R du composé halogéné de formule générale (II) désigne une chaîne hydrocarbonée saturée ou non, linéaire ou cyclique ayant de 1 à 24, préférentiellement de 1 à 10 chaînons carbonés et tout particulièrement un atome de carbone. Cette chaîne peut être substituée par un ou plusieurs radicaux hydrocarbonés, saturés, linéaires ou cycliques ayant de 1 à 12 atomes de carbones, avantageusement de 1 à 6 tels que méthyle, éthyle, propyle, isopropyle, butyle, isobutyle, pentyle, néopentyle, n-hexyle et tout particulièrement méthyle.

Avantageusement, le dérivé de 1,3-diphénylprop-2-èn-1-one substitué par un groupement hydroxyle ou thiol utilisé dans le cadre de l'étape (i) précédemment décrite est obtenu par une réaction de Claisen Schmidt en milieu acide ou basique d'un composé de type acétophénone avec un dérivé thio- ou hydroxy-benzaldéhyde, ou d'un dérivé de thio- ou hydroxy-acétophénone avec un dérivé de type benzaldéhyde.

Le procédé selon l'invention comprend, ultérieurement à l'étape (i) telle que décrite ci-dessus, une étape appelée (ii) de préparation du dérivé de carboxyalkyloxy ou carboxyalkylthio de 1,3-diphénylprop-2-èn-1-one par réaction d'acidolyse de l'ester obtenu à l'étape (i) avec de l'acide trifluoroaétique.

Cettte étape (ii) peut être résumée selon l'un des schémas réactionnels suivants : 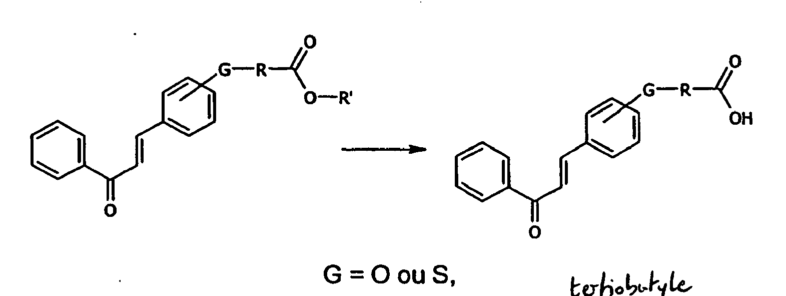
R = chaîne alkyle et R' = groupement tertiobutyle
Etape (ii) : première illustration

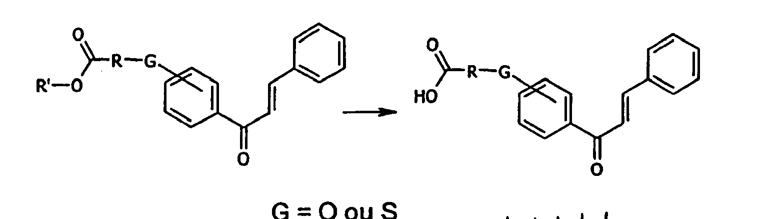

R = chaîne alkyle et R' = groupement tertiobutyle
Etape (ii) : seconde illustration

Cette étape d'acidolyse est réalisée en mettant en contact un dérivé de 1,3-diphénylprop-2-èn-1-one substitué par un groupement alkyloxycarbonylalkyloxy ou alkyloxycarbonylalkylthio avec de l'acide trifluoroacétique. Généralement, la quantité d'acide trifluoroacétique est de 1 à 20 équivalents, et préférentiellement de 8 à 12 équivalents. Cette étape est avantageusement réalisée à une température de 0 à 100°C et plus préférentiellement de 18 à 25°C, et préférentiellement à pression atmosphérique.

La présente invention décrit donc la préparation de 1,3-diphénylprop-2-èn-1-ones substituées par un groupement carboxyalkyloxy ou un groupement carboxyalkylthio. L'intérêt de cette méthode réside dans la combinaison de deux étapes de synthèse : synthèse d'esters tertiobutyliques à partir de 1,3-diphénylprop-2-èn-1-ones substituées sur l'un des groupes phényl par un groupement hydroxy ou thiol suivie de la réaction d'acydolyse des esters intermédiairement préparés avec l'acide trifluoroacétique.

Le groupement tertiobutyloxycarbonylalkyle est introduit facilement, avec un rendement élevé par réaction d'alkylation d'un précurseur chimique (hydroxy-1,3-diphénylprop-2-èn-1-one ou son équivalent soufré) avec un dérivé halogéné. L'ester de tertiobutyle intermédiaire obtenu peut être purifié facilement, notamment par chromatographie sur gel de silice ou par recristallisation.

L'ester de tertiobutyle est clivé en l'acide correspondant par action d'acide trifluoroacétique. Cette méthode, adaptée au clivage du groupement tertiobutyle permet d'obtenir la transformation totale de l'ester en l'acide correspondant. Les inventeurs ont découvert que cette méthode est compatible avec la structure chimique des 1,3-diphénylprop-2-én-1-ones. Par conséquent, elle ne provoque pas l'apparition de produits de dégradation et permet d'obtenir les acides avec des rendements plus élevés que ceux observés classiquement.

Les trois étapes pouvant être mises en oeuvre dans le cadre de la présente invention peuvent être résumées de la manière suivante.

### Première étape : synthèse du précurseur chimique :

Le précurseur chimique de type hydroxy 1,3-diphénylprop-2-èn-1-one ou son analogue soufré peut être préparé par la réaction classique de Claisen Schmidt en milieu acide ou basique :

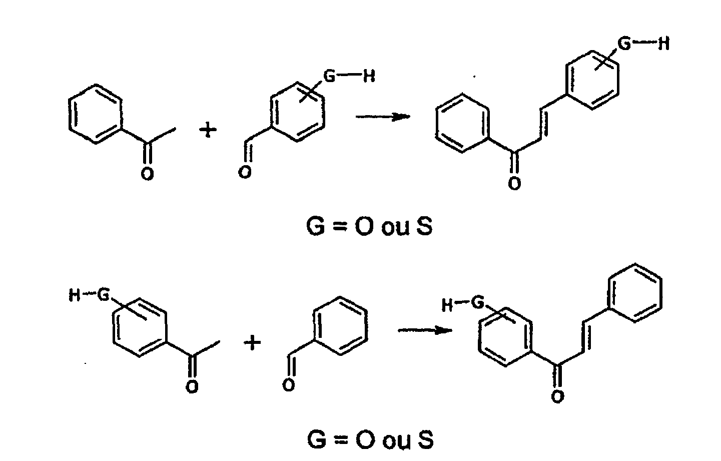

Les composés de type acétophénone, hydroxy-acétophénone (ou son analogue soufré), benzaldéhyde et hydroxy-benzaldéhyde (ou son analogue soufré) mis en oeuvre dans cette réaction peuvent éventuellement être substitués sur les groupes phényles. Ces substituants sont plus particulièrement choisis parmi un atome d'halogène, un radical alkyle, un groupement thionitroso, et un radical alkyloxy ou alkylthio.

Les conditions de mise en oeuvre de cette réaction en milieu acide ou basique sont à la portée de l'homme du métier et peuvent varier dans une large mesure.

La mise en contact de ces deux composés est avantageusement réalisée de manière stoechiométrique. Elle est réalisée de préférence à une température ambiante (entre environ 18°C et 25°C) et à pression atmosphérique.

En milieu basique, la réaction est de préférence réalisée en présence d'une base forte, tel qu'un hydroxyde de métal alcalin, comme l'hydroxyde de sodium,
ou un alcoolate de métal alcalin tel que l'éthylate de sodium.

En milieu acide, la réaction est de préférence réalisée en présence d'un acide fort, tel que l'acide chlorhydrique.

La synthèse en milieu basique peut être avantageusement réalisée de la façon suivante :

La cétone à 1 équivalent-molaire et l'aldéhyde à 1 équivalent-molaire sont solubilisés dans une solution hydroalcoolique d'hydroxyde de sodium à 20 équivalents-molaire. L'ensemble est agité pendant 6 à 48 heures et préférentiellement 16 à 20 heures à une température de 0 à 100°C et préférentiellement 18 à 25°C. Le milieu est ensuite acidifié (pour atteindre en particulier un pH d'environ 2), notamment avec de l'acide chlorhydrique.
L'hydroxy 1,3-diphénylprop-2-èn-1-one (ou son analogue soufré) attendue peut être obtenue par précipitation ou extraction solide/liquide après évaporation du milieu réactionnel. Elle peut être ensuite purifiée par chromatographie sur gel de silice ou par recristallisation.

La synthèse en milieu acide peut être avantageusement réalisée de la façon suivante :

La cétone à 1 équivalent-molaire et l'aldéhyde à 1 équivalent-molaire sont solubilisés dans une solution d'éthanol saturée d'acide chlorhydrique gazeux. L'ensemble est maintenu sous agitation à une température de 0 à 100°C et préférentiellement à une température de 18 à 25°C pendant 1 à 24 heures et préférentiellement 1 à 6 heures, de préférence à pression atmosphérique. Le solvant est éliminé notamment par évaporation sous pression réduite. La 1,3-diphénylprop-2-èn-1-one est purifiée, notamment par chromatographie sur gel de silice.

### Seconde étape : préparation de l'ester de tertiobutyle

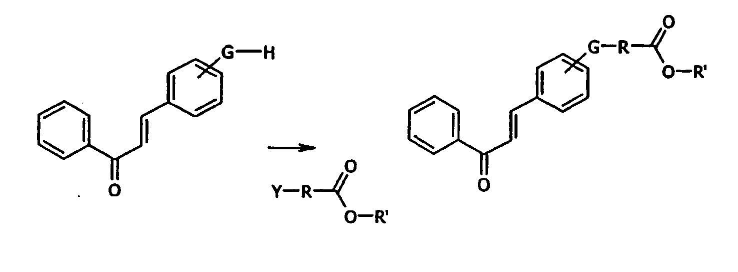

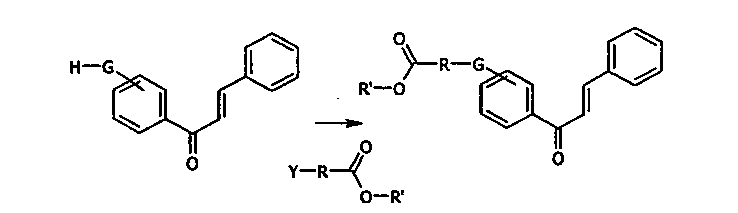

G représentant un atome d'oxygène ou de soufre,
Y représentant un atome d'halogène,
R est un chaîne alkyle, de préférence de C1 à C6
R' = tertiobutyle

La réaction peut être réalisée de la façon suivante :
L'hydroxy 1,3-diphénylprop-2-èn-1-one (ou l'analogue soufré) à 1 équivalent molaire est solubilisée dans un solvant, préférentiellement dans de l'acétonitrile ou de l'acétone. 1 à 10 équivalents et préférentiellement 4 à 6 équivalents de carbonate de potassium ou de césium sont ajoutés puis est ajouté le dérivé de type halogénoester de tertiobutyle (1 à 20 équivalents molaires et préférentiellement 4 à 8 équivalents molaires et encore plus préférentiellement 6 équivalents). L'ensemble est chauffé sous (vive) agitation à une température de 25 à 120°C et préférentiellement de 100°C pendant 1 à 24 heures et préférentiellement 10 à 14 heures et encore plus préférentiellement 10 heures, en général à pression atmosphérique. Le solvant est éliminé, notamment par évaporation sous pression réduite. Le milieu réactionnel est éventuellement remis en réaction avec 3 à 6 équivalents-molaire de dérivé halogéné et 3 à 5 équivalents-molaire de carbonate de potassium ou de césium, cette opération pouvant être réitérée jusque disparition complète de la matière première.
La 1,3-diphénylprop-2-èn-1-one substituée par un groupement tertiobutyloxycarbonylalkyloxy, tertiobutyloxycarbonylalkylthio, isopropyloxycarbonylalkyloxy, ou isopropyloxycarbonylalkylthio, est purifiée, notamment par chromatographie sur gel de silice.

### Troisième étape : préparation de l'acide à partir de l'ester tertiobutylique :

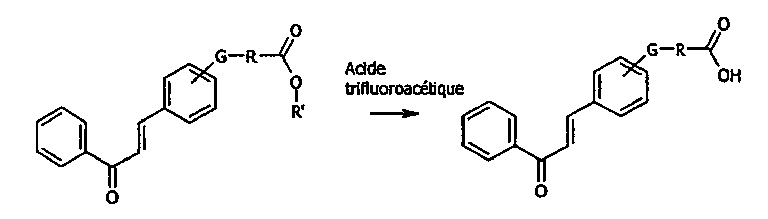

G et R sont tels que définis précédemment.
R' = tertiobutyle

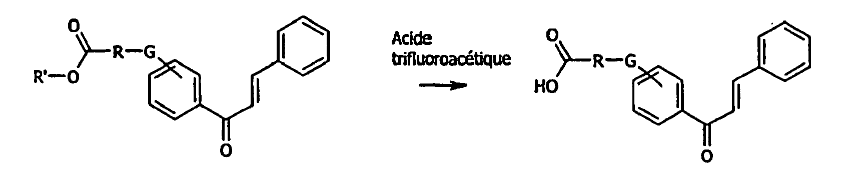

G et R sont tels que définis précédemment.
R' = tertiobutyle

La 1,3-diphénylprop-2-èn-1-one substituée par un groupement tertiobutyloxycarbonylalkyloxy, par un groupement tertiobutyloxycarbonylalkylthio, à 1 équivalent molaire est solubilisée dans un solvant, tel que du dichlorométhane. 1 à 20 équivalents et préférentiellement 8 à 12 équivalents d'acide et plus préférentiellement 10 équivalents d'acide, l'acide étant l'acide trifluoroacétique, sont ajoutés. L'ensemble est maintenu sous vive agitation à une température de 0 à 100°C, préférentiellement de 18 à 25°C, pendant 1 à 24 heures et préférentiellement 8 à 14 heures et plus préférentiellement 12 heures, de préférence à pression atmosphérique. Le solvant est éliminé, notamment par évaporation sous pression réduite. La 1,3-diphénylprop-2-èn-1-one substituée par un groupement carboxyalkyloxy ou carboxyalkylthio est purifiée, notamment par chromatographie sur gel de silice. La réaction est réalisée de préférence à pression atmosphérique.

Ce procédé peut avantageusement être mis en oeuvre pour la préparation des composés décrits dans la demande de brevet français n° FR 02 08571 déposée par la Demanderesse le 8 juillet 2002. Ces composés répondent à la formule générale : 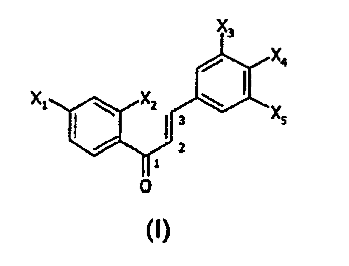 dans laquelle:
X1 représente un halogène ou un groupement -R1 ou un groupement répondant à la formule suivante : -G1-R1 ;
X2 représente un atome d'hydrogène ou un groupement thionitroso ou un groupement alkyloxy ou un groupement alkylcarbonyloxy ou un groupement alkylthio ou un groupement alkylcarbonylthio ;
X3 représente un groupement -R3 ou un groupement répondant à la formule suivante : -G3-R3 ;
X4 représente un halogène ou un groupement thionitroso ou un groupement -R4 ou un groupement répondant à la formule suivante : -G4-R4 ;
X5 représente un groupement -R5 ou un groupement répondant à la formule suivante : -G5-R5 ;
R1, R3, R4, R5, identiques ou différents, représentent un atome d'hydrogène ou un groupement alkyle substitué ou non par une fonction acide carboxylique ;
G1, G3, G4, G5, identiques ou différents, représentent un atome d'oxygène ou de soufre ;
avec un des groupements X1, X3, X4 ou X5 répondant à la formule -G₁-R₁, -G₃-R₃, -G₄-R₄ ou -G₅-R₅, dans laquelle R₁, R₃, R₄ ou R₅ respectivement est un radical alkyle portant une fonction acide carboxylique.

Ce procédé de synthèse permet avantageusement de préparer les composés de la formule générale (I) dans laquelle aucun des groupements X1, X2, X3 et X4 n'est un groupement hydroxy ou thiol.

De manière préférentielle, ce procédé permet de préparer les composés de formule générale (I) pour lesquels au moins un des groupements R3, R4 et R5 représente un radical alkyle portant une fonction acide carboxylique.

De manière préférentielle, R4 est un radical alkyle portant une fonction acide carboxylique.

De manière préférentielle, R4 est un radical alkyle portant une fonction acide carboxylique et X3 et X5 sont des alkyles non substitués.

De manière plus préférentielle, X4 est un groupement carboxydiméthylméthyloxy ou carboxydiméthylméthylthio.

De manière préférentielle, G4 est un atome d'oxygène et R4 est un radical alkyle portant une fonction acide carboxylique

De manière préférentielle, G4 est un atome d'oxygène et R4 est un radical alkyle portant une fonction acide carboxylique et X3 et X5 sont des alkyles non substitués.

De manière plus préférentielle, X4 est un groupement carboxydiméthylméthyloxy.

De manière plus préférentielle, X4 est un groupement carboxydiméthylméthylthio.

Les dérivés de formule générale (I) tels que décrits ci-dessus peuvent adopter la conformation cis ou trans.

De manière avantageuse, X3, X4 et X5 ne sont pas des atomes d'hydrogène.

Selon la présente invention, le terme "alkyle" désigne un radical hydrocarboné saturé, linéaire, ramifié ou cyclique, halogéné ou non, ayant plus particulièrement de 1 à 24, de préférence 1 à 10, atomes de carbone tels que méthyle, éthyle, propyle, isopropyle, butyle, isobutyle, *tertio*butyle, pentyle, néopentyle, n-hexyle. Les groupes en C₁-C₂ ou C₂-C₇ sont particulièrement préférés. Les groupes méthyle et éthyle sont tout particulièrement préférés.

Le terme thionitroso fait référence à un groupement nitroso lié au cycle aromatique par l'intermédiaire d'un atome de soufre.

Le terme halogène représente un atome de chlore ou un atome de brome ou un atome d'iode ou un atome de fluor.

Le terme alkyloxy fait référence à une chaîne alkyle liée au cycle par l'intermédiaire d'un atome d'oxygène. La chaîne alkyle répond à la définition précédemment énoncée.

Le terme alkylthio fait référence à une chaîne alkyle liée au cycle aromatique par l'intermédiaire d'un atome de soufre (liaison thioéther). La chaîne alkyle répond à la définition précédemment énoncée.

Les composés ou produits intermédiaires (esters) préférentiellement obtenus par le procédé selon l'invention sont indiqués ci-dessous avec les formules qui leur sont associées :
le 1-[4-chlorophényl]-3-[3,5-diméthyl-4-tertiobutyloxycarbonyldiméthylméthyloxyphényl]prop-2-èn-1-one 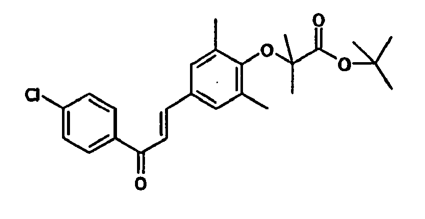
le 1-[4-chlorophényl]-3-[3,5-diméthyl-4-carboxydiméthylméthyloxyphényl]prop-2-èn-1-one 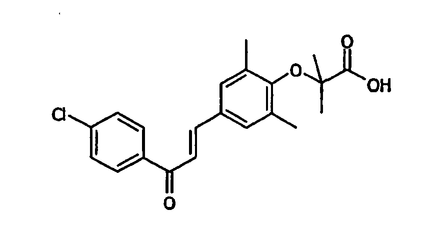
le 1-[4-méthylthiophényl]-3-[4-carboxydiméthylméthyloxyphényl]prop-2-èn-1-one 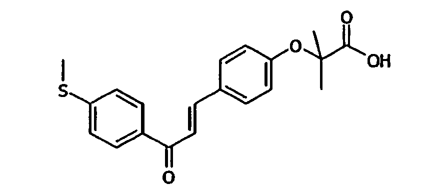
le 1-[4-carboxydiméthylméthyloxyphényl]-3-[4-chlorophényl]prop-2-èn-1-one 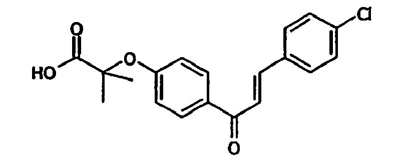
le 1-[4-carboxydiméthylméthylthiophényl]-3-[4-méthylthiophényl]prop-2-èn-1-one 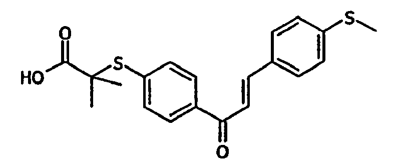
le 1-[4-carboxydiméthylméthyloxyphényl]-3-[4-méthytthiophényl]prop-2-èn-1-one 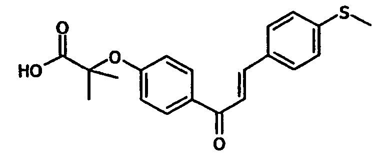
le 1-[4-méthylthiophényl]-3-[3,5-diméthyl-4-tertiobutyloxycarbonyldiméthylméthyloxyphényl]prop-2-èn-1-one 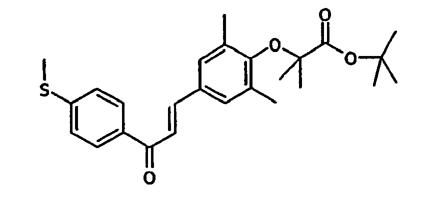
le 1-[4-méthylthiophényl]-3-[3,5-diméthyl-4-carboxydiméthylméthyloxyphényl]prop-2-èn-1-one 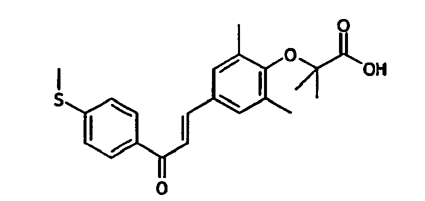
le 1-[2-méthoxyphényl]-3-[3,5-diméthyl-4-tertiobutyloxycarbonyldiméthylméthyloxyphényl]prop-2-èn-1-one 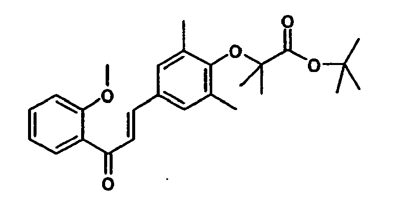
le 1-[2-méthoxyphényl]-3-[3,5-diméthyl-4-carboxydiméthylméthyloxyphényl]prop-2-èn-1-one 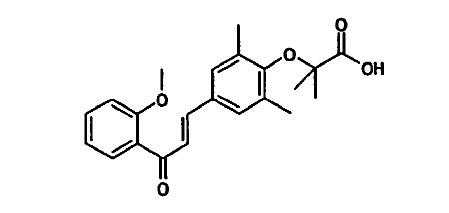
le 1-[4-hexyloxyphényl]-3-[3,5-diméthyl-4-tertiobutyloxycarbonyldiméthylméthyloxyphényl]prop-2-èn-1-one 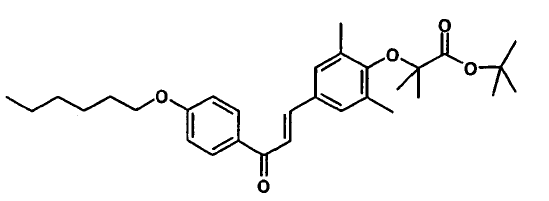
le 1-[4-hexyloxyphényl]-3-[3,5-diméthyl-4-carboxydiméthylméthyloxyphényl]prop-2-èn-1-one 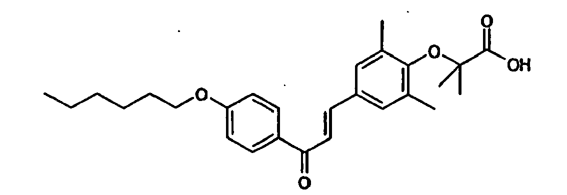
le 1-[2-méthyloxy-4-chlorophényl]-3-[3,5-diméthyl-4-tertiobutyloxycarbonyldiméthylméthyloxyphényl]prop-2-èn-1-one 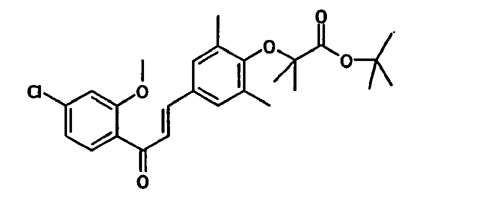
le 1-[2-méthyloxy-4-chlorophényl]-3-[3,5-diméthyl-4-carboxydiméthylméthyloxyphényl]prop-2-èn-1-one 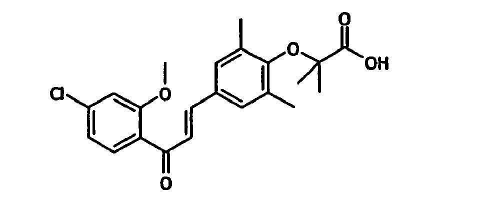
le 1-[4-heptylphényl]-3-[3,5-diméthyl-4-tertiobutyloxycarbonyldiméthylméthyloxyphényl]prop-2-èn-1-one 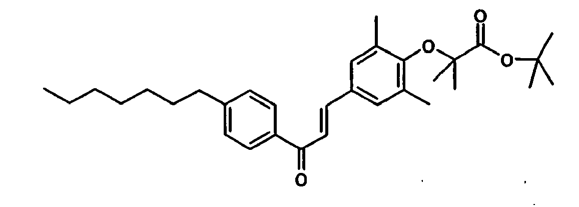
le 1-[4-heptylphényl]-3-[3,5-diméthyl-4-carboxydiméthylméthyloxyphényl]prop-2-èn-1-one 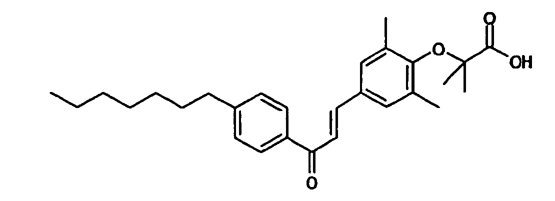
le 1-[4-bromophényl]-3-[3,5-diméthyl-4-tertiobutyloxycarbonyldiméthylméthyloxyphényl]prop-2-èn-1-one 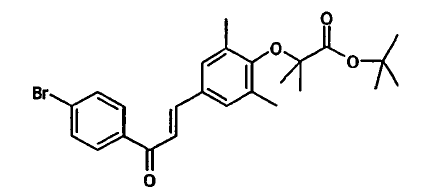
le 1-[4-bromophényl]-3-[3,5-diméthyl-4-carboxy diméthylméthyloxyphényl]prop-2-èn-1-one

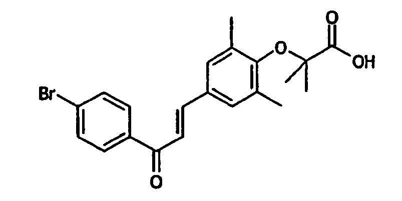

D'autres aspects et avantages de la présente invention apparaîtront à la lecture des exemples qui suivent, qui doivent être considérés comme illustratifs.

### EXEMPLES

### 1/Description des méthodes générales de synthèse :

### Méthode générale 1 :

### Synthèse des hydroxy-1,3-diphénylprop-2-èn-1-ones :

L'hydroxy-acétophénone (ou son analogue soufré) à 1 équivalent-molaire et l'aldéhyde à 1 équivalent-molaire, ou l'hydroxy-benzaldéhyde (ou son analogue soufré) à 1 équivalent-molaire et la cétone à 1 équivalent-molaire, sont solubilisés dans une solution d'éthanol saturée d'acide chlorhydrique gazeux. L'ensemble est agité à température ambiante pendant 1 à 6h puis le solvant est éliminé par évaporation sous pression réduite. L'hydroxy-1,3-diphénylprop-2-èn-1-one est purifiée par chromatographie sur gel de silice ou par recristallisation.

### Méthode générale 2 :

### Alkylation des hydroxy-1,3-diphénylprop-2-èn-1-ones :

L' hydroxy-1,3-diphénylpropèn-1-one (ou son analogue soufré) à 1 équivalent-molaire est solubilisée dans l'acétonitrile et le dérivé halogéné 3 à 6 équivalents-molaire puis le carbonate de potassium 3 à 5 équivalents-molaire sont ajoutés. Le milieu réactionnel est maintenu environ 10 heures sous vive agitation à reflux. Les sels sont éliminés par filtration.
Le milieu réactionnel est éventuellement remis en réaction avec 3 à 6 équivalents-molaire de dérivé halogéné et 3 à 5 équivalents-molaire de carbonate de potassium. Cette opération peut être réitérée jusqu'à complète disparition de la matière première.
Le solvant et l'excès de réactif sont éliminés par évaporation sous pression réduite, le produit attendu est purifié par chromatographie sur gel de silice.

### Méthode générale 3 :

Acidolyse d'esters tertiobutyliques de 1,3-diphénylprop-2-èn-1-ones par l'acide trifluoroacétique :

L'ester tertiobutylique de 1,3-diphénylprop-2-èn-1-one à 1 équivalent-molaire est solubilisé dans du dichlorométhane, l'acide trifluoroacétique à 10 équivalents-molaire est additionné, l'ensemble est maintenu 12 heures sous agitation à température ambiante. Le produit formé est purifié par chromatographie sur gel de silice ou par recristallisation.

### 2/ Exemples :

### Exemple 1 : synthèse de la 1-[4-chlorophényl]-3-[3,5-diméthyl-4-carboxydiméthylméthyloxyphényl]prop-2-èn-1-one :

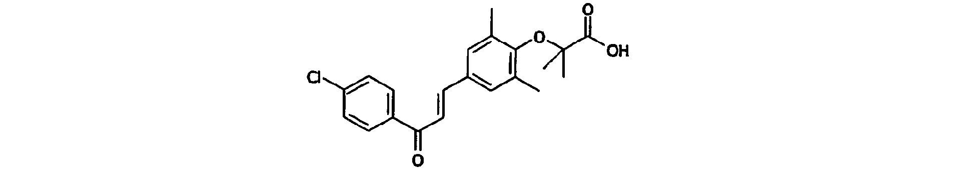

Le procédé permet la préparation de ce composé avec un rendement global de 56%.

### Synthèse du précurseur chimique :

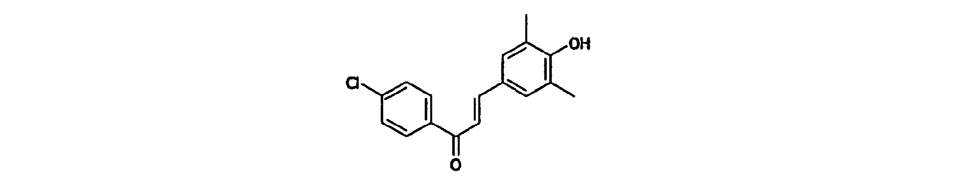

### 1-[4-chlorophényl]-3-[3,5-diméthyl-4-hydroxyphényl]prop-2-èn-1-one (Composé 1)

Ce composé a été synthétisé à partir de 4-chloroacétophénone et de 3,5-diméthyl-4-hydroxybenzaldéhyde selon la méthode générale 1 précédemment décrite. Purification par chromatographie sur gel de silice (cyclohexane/acétate d'éthyle : 95/5). Rendement : 91%
RM N 1H CDCl₃ δ ppm : 2.30(s, 6H), 7.32(s, 2H), 7.34(d, J = 15.25Hz, 1H), 7.47(d, J = 8.86Hz, 2H), 7.75(d, J = 15.26, 1H), 7.97(d, J = 8.86Hz, 2H).

### Synthèse de l'ester de tertiobutyle :

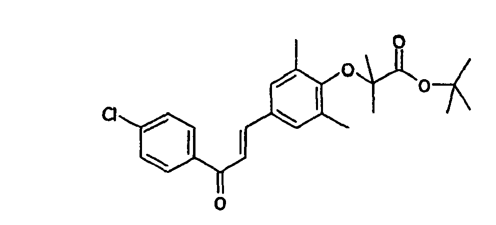

### 1-[4-chlorophényl]-3-[3,5-diméthyl-4-tertiobutyloxycarbonyldiméthylméthyloxyphényl]prop-2-èn-1-one (Composé 2)

Ce composé a été synthétisé à partir du 1-[4-chlorophényl]-3-[3,5-diméthyl-4-hydroxyphényl]prop-2-èn-1-one (Composé 1) et de bromoisobutyrate de tertiobutyle selon la méthode générale 2 précédemment décrite.
Purification par chromatographie sur gel de silice (cyclohexane/acétate d'éthyle : 90/10). Rendement: 70 %

### Acidolyse de l'ester de tertiobutyle :

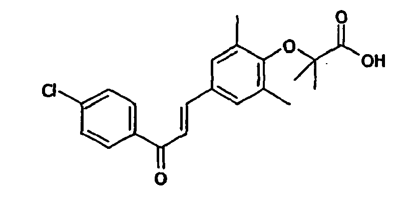

### 1-[4-chlorophényl] -3-[3,5-diméthyl-4-carboxydiméthylméthyloxyphényl]prop-2-èn-1-one (Composé 3).

Ce composé a été synthétisé à partir de 1-[4-chlorophényl]-3-[3,5-diméthyl-4-tertiobutyloxycarbonyldiméthylméthyloxyphényl]prop-2-èn-1-one (composé 2) selon la méthode générale 3 précédemment décrite.
Purification par chromatographie sur gel de silice (dichlorométhane/méthanol: 98/2). Rendement : 88%
RMN 1H DMSO δ ppm : 1.39(s, 6H), 2.22(s, 6H), 7.58(s, 2H), 7.67-7.62(m, 3H), 7.82(d, J = 15,5 Hz, 1H), 8.17(d, 2H), 12.96(s, 1H)
SM(Maldi-Tof): 373.3(M+1)

### Exemple 2 : synthèse du 1-[2-méthoxyl-3-[3,5-diméthyl-4-carboxydiméthylméthyloxyphényl]prop-2-èn-1-one :

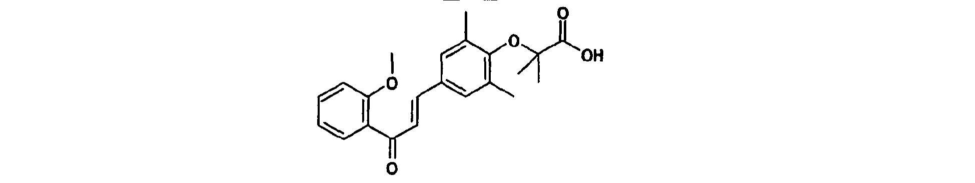

Le procédé permet la préparation de ce composé avec un rendement global de 20%.

### Synthèse du précurseur chimique :

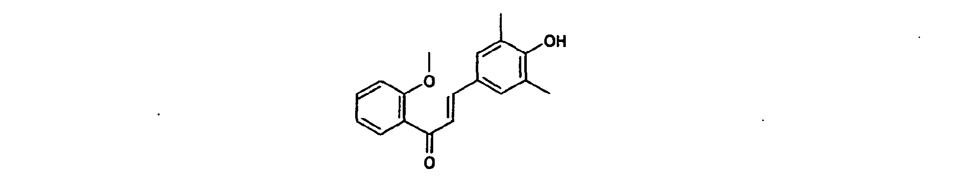

### 1-[2-méthoxyphényl] -3-[3,5-diméthyl-4-hydroxyphényl]prop-2-èn-1-one (Composé 4)

Ce composé a été synthétisé à partir de 2-méthoxyacétophénone et de 3,5-diméthyl-4-hydroxybenzaldéhyde selon la méthode générale 1 précédemment décrite.
Purification par chromatographie sur gel de silice (cyclohexane/acétate d'éthyle : 80/20). Rendement : 66%
RMN 1H CDCl3 δ ppm : 2,27(s, 6H), 3,87(s, 3H), 6,97-7,05(m, 2H), 7,19(d, 1H, J = 15,96 Hz), 7,22(s, 2H), 7,44(m, 1H), 7,51 (d, 1H, J = 15,48 Hz), 7,56 (dd, 1H, J = 1,86 Hz, J = 7,5 Hz)

### Synthèse de l'ester de tertiobutyle :

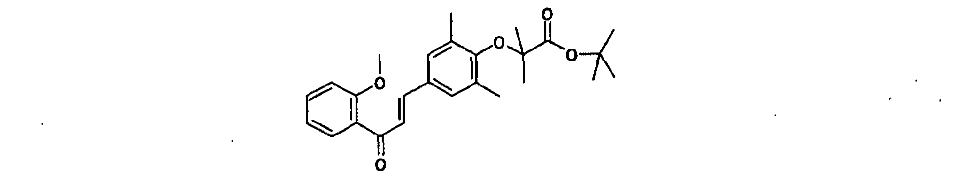

### 1-[2-méthoxyphényl]-3-[3,5-diméthyl-4-tertiobutyloxycarbonyldiméthylméthyloxyphényl]prop-2-èn-1-one (Composé 5)

Ce composé a été synthétisé à partir de 1-[2-méthoxyphényl]-3-[3,5-diméthyl-4-hydroxyphényl]prop-2-èn-1-one (Composé 4) et de bromoisobutyrate de tertiobutyle selon la méthode générale 2 précédemment décrite.
Purification par chromatographie sur gel de silice (cyclohexane/acétate d'éthyle : 80/20). Rendement : 41%

### Acidolyse de l'ester de tertiobutyle :

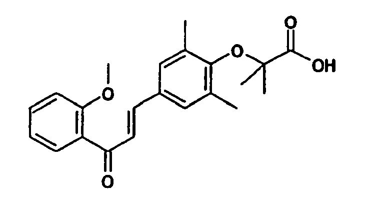

### 1-[2-méthoxyphényl] -3-[3,5-diméthyl-4-carboxydiméthylméthyloxyphényl]prop-2-èn-1-one (Composé 6)

Ce composé a été synthétisé à partir de 1-[2-méthoxyphényl]-3-[3,5-diméthyl-4-tertiobutyloxycarbonyldiméthylméthyloxyphényl]prop-2-èn-1-one (Composé 5) selon la méthode générale 3 précédemment décrite.
Purification par chromatographie sur gel de silice (dichlorométhane/méthanol : 98/2). Rendement : 70%
RMN 1H DMSO δ ppm: 1.38(s, 6H) , 2.19(s, 6H), 3.93(s, 3H), 7.05(m, 1H), 7.20(d, J = 8.31 Hz, 1H), 7.25(d, J = 15.5Hz, 1H), 7.37(d, J = 15.5Hz, 1H), 7.39(s, 2H), 7.46(d, J = 7.2Hz, 1H), 7.53(m, 1H), 12.93(s, 1H)
SM(ES-MS) : 367.1(M-1)

### Exemple 3 :

### Synthèse de la 1-[4-méthylthiophényl] -3-[3,5-diméthyl-4-carboxydiméthylméthyloxyphényl]prop-2-èn-1-one :

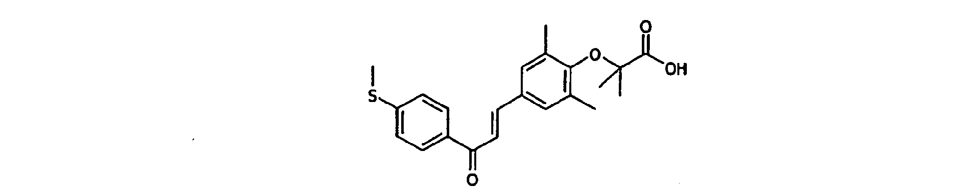

Le procédé permet la préparation de ce composé avec un rendement global de 49%.

### Synthèse du précurseur chimique :

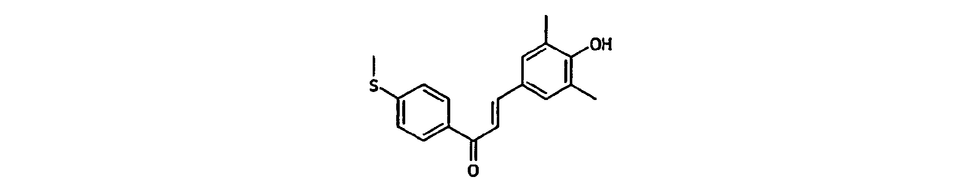

### 1-[4-méthylthiophényl] -3-[3,5-diméthyl-4-hydroxyphényl]prop-2-èn-1-one (Composé 7)

Ce composé a été synthétisé à partir de 4-méthylthioacétophénone et de 3,5-diméthyl-4-hydroxybenzaldéhyde selon la méthode générale 1 précédemment décrite.
Purification par chromatographie sur gel de silice (cyclohexane/acétate d'éthyle : 80/20). Rendement : 86%
RMN 1H DMSO δ ppm : 1.22(s, 6H), 2.54(s, 3H), 7.36(d, J = 8.20Hz, 2H), 7.48(s, 2H), 7.62(d, J = 15.7Hz, 1H), 7.74(d, J = 15.7Hz, 1H), 8.10(d, J = 8.20Hz, 2H), 8.92(s, 1H)

### Synthèse de l'ester de tertiobutyle :

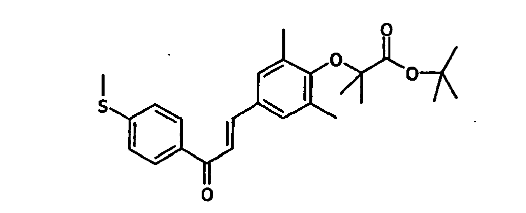

### 1-[4-méthylthiophényl]-3-[3,5-diméthyl-4-tertiobutyloxycarbonyldiméthylméthyloxyphényl]prop-2-èn-1-one (Composé 8)

Ce composé a été synthétisé à partir de 1-[4-méthylthiophényl]-3-[3,5-diméthyl-4-hydroxyphényl]prop-2-èn-1-one (Composé 7) et de bromoisobutyrate de tertiobutyle :
La 1-[4-méthylthiophényl]-3-[3,5-diméthyl-4-hydroxyphényl]prop-2-èn-1-one est solubilisée dans l'acétonitrile puis le carbonate de potassium à 3 équivalents-molaire et le bromoisobutyrate de tertiobutyle à 3 équivalents-molaire sont ajoutés. L'ensemble est maintenu sous agitation à 80°C pendant 12h00, puis est ramené à température ambiante. Les sels sont éliminés par filtration. 3 équivalents-molaire de carbonate de potassium et 3 équivalents-molaire de bromoisobutyrate de tertiobutyle sont ajoutés. L'ensemble est remis en réaction pendant 12 h00 puis est ramené à température ambiante. Les sels sont éliminés par filtration. 3 équivalents-molaire de carbonate de potassium et 3 équivalents-molaire de bromoisobutyrate de tertiobutyle sont ajoutés. L'ensemble est maintenu sous agitation à 80°C pendant 12h00. Les sels sont éliminés par filtration, le solvants sont éliminés par évaporation sous pression réduite.
Purification par chromatographie sur gel de silice (cyclohexane/acétate d'éthyle : 90/10). Rendement : 74%

### Acidolyse de l'ester de tertiobutyle :

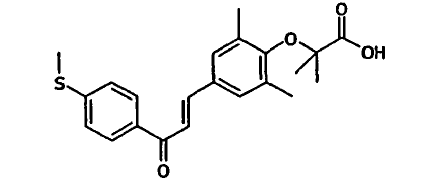

### 1-[4-méthylthiophényl] -3-[3,5-diméthyl-4-carboxydiméthylméthyloxyphényl]prop-2-èn-1-one (Composé 9)

Ce composé a été synthétisé à partir de 1-[4-méthylthiophényl]-3-[3,5-diméthyl-4-tertiobutyloxycarbonyldiméthylméthyloxyphényl]prop-2-èn-1-one (Composé 8) selon la méthode générale 3 précédemment décrite.
Purification par chromatographie sur gel de silice (dichlorométhanelméthanol : 98/2) Rendement : 81%
RMN 1H DMSO δ ppm : 1.39(s, 6H), 2.22(s, 6H), 2.57(s, 3H), 7.40(d, J = 8.55Hz, 2H), 7.57(s, 2H), 7.62(d, J = 15.5Hz, 1H), 7.83(d, J = 15.5Hz, 1H), 8.10(d, J = 8.55Hz, 2H), 12.97(s, 1H)
SM(ES-MS): 383.3(M-1)

### Exemple 4 :

### Synthèse de la 1-[4-hexyloxyphényl] -3-[3,5-diméthyl-4-carboxydiméthylméthyloxyphény]prop-2-èn-1-one :

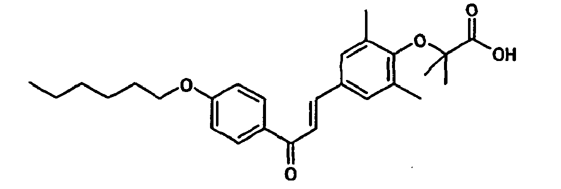

Le procédé permet la préparation de ce composé avec un rendement global de 24%.

### Synthèse du précurseur chimique :

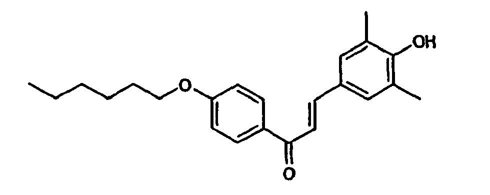

### 1-[4-hexyloxyphényl] -3-[3,5-diméthyl-4-hydroxyphényl]prop-2-èn-1-one (Composé 10)

Ce composé a été synthétisé à partir de 4-hexyloxyacétophénone et de 3,5-diméthyl-4-hydroxybenzaldéhyde selon la méthode générale 1 précédemment décrite. Le produit cristallise en fin de réaction, il est essoré. Rendement : 63%
RM N 1H DMSO δ ppm : 0.88(m, 3H), 1.28-1.43(m, 6H), 1.72(m, 2H), 2.21 (s, 6H), 4.05(t, J = 6.42Hz, 2H), 7.40(d, J = 8.43Hz, 2H), 7.48(s, 2H), 7.57(d, J = 15.24Hz, 1H), 7.72(d, J = 15.24Hz, 1H), 8.12(d, J = 8.43Hz, 2H), 8.89(s, 1H)

### Synthèse de l'ester de tertiobutyle :

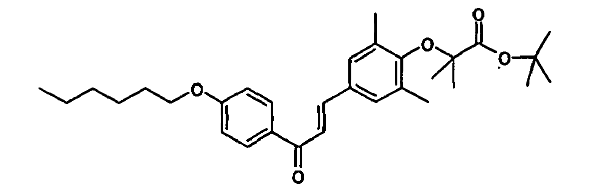

### 1-[4-hexyloxyphényl] -3-[3,5-diméthyl-4-tertiobutyloxycarbonyldiméthylméthyloxyphényl]prop-2-èn-1-one (Composé 11)

Ce composé a été synthétisé à partir de 1-[4-hexyloxyphényl]-3-[3,5-diméthyl-4-hydroxyphényl]prop-2-èn-1-one (Composé 10) et de bromoisobutyrate de tertiobutyle selon la méthode générale 2 précédemment décrite.
Purification par chromatographie sur gel de silice (cyclohexane/acétate d'éthyle : 95/5). Rendement : 75%

### Acidolyse de l'ester de tertiobutyle :

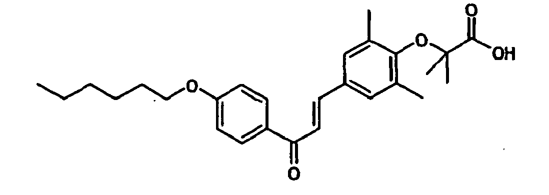

### 1-[4-hexyloxyphényl] -3-[3,5-diméthyl-4-carboxydiméthylméthyloxyphényl]prop-2-èn-1-one (Composé 12)

Ce composé a été synthétisé à partir de 1-[4-héxyloxyphényl]-3-[3,5-diméthyl-4-tertiobutyloxycarbonyldiméthylméthyloxyphényl]prop-2-èn-1-one (Composé 11) selon la méthode générale 3 précédemment décrite.
Purification par recristallisation dans le méthanol. Rendement : 51 %
RMN 1H DMSO δ ppm : 0.88(t, J = 6.33Hz, 3H), 1.30(m, 4H), 1.39(s, 6H), 1.44(m, 2H), 1.73(m, 2H), 2.22(s, 6H), 4.06(t, J = 6.30Hz, 2H), 7.06(d, J = 8.61 Hz, 2H), 7.56(s, 2H), 7.58(d, J = 15.5Hz, 1H), 7.82(d, J = 15.5Hz, 1H), 8.13(d, J = 6.61 Hz, 2H)
SM(ES-MS): 437.2(M-1)

### Exemple 5 :

### Synthèse de la 1-[2-méthyloxy-4-chlorophényl] -3-[3,5-diméthyl-4-carboxydiméthylméthyloxyphényl]prop-2-èn-1-one

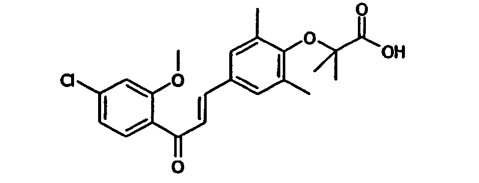

Le procédé permet la préparation de ce composé avec un rendement global de 22%.

### Synthèse du précurseur chimique :

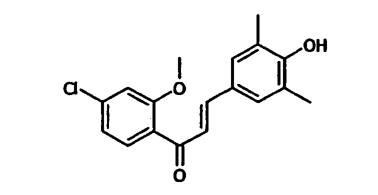

### 1-[2-méthyloxy-4-chlorophényl] -3-[3,5-diméthyl-4-hydroxyphényl]prop-2-èn-1-one (Composé 13)

Ce composé a été synthétisé à partir de 4-chloro-2-méthoxyacétophénone et de 3,5-diméthyl-4-hydroxybenzaldéhyde selon la méthode générale 1 précédemment décrite.
Purification par chromatographie sur gel de silice (cyclohexane/acétate d'éthyle : 85/15). Rendement : 72%
RMN 1H DMSO δ ppm : 2.21 (s, 6H), 3.90(s, 3H), 7.12(m, 1H), 7.23(d, J = 15.5Hz, 1H), 7.29(d, J = 1.80Hz, 1H), 7.38(d, J = 15.5 Hz, 1H), 7.41(s, 2H), 7.48(d, J = 7.98Hz, 1H)

### Synthèse de l'ester de tertiobutyle :

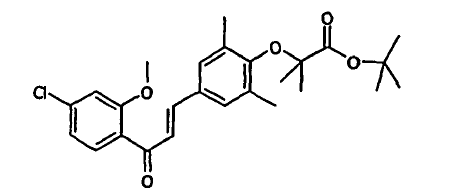

### 1-[2-méthyloxy-4-chlorophényl] -3-[3,5-diméthyl-4-tertiobutyloxycarbonyldiméthylméthyloxyphényl]prop-2-èn-1-one (Composé 14)

Ce composé a été synthétisé à partir de 1-[2-méthyloxy-4-chlorophényl]-3-[3,5-diméthyl-4-hydroxydiméthylméthyloxyphényl]prop-2-èn-1-one (Composé 13) et de bromoisobutyrate de tertiobutyle selon la méthode générale 2 précédemment décrite.
Purification par chromatographie sur gel de silice (cyclohexane/acétate d'éthyle : 90/10). Rendement : 43%

### Acidolyse de l'ester de tertiobutyle :

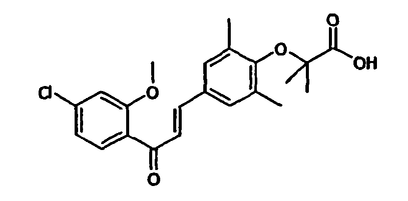

### 1-[2-méthyloxy-4-chlorophényl] -3-[3,5-diméthyl-4-carboxydiméthylméthyloxyphényl]prop-2-èn-1-one (Composé 15)

Ce composé a été synthétisé à partir de 1-[2-méthoxy-4-chlorophényl]-3-[3,5-diméthyl-4-tertiobutyloxycarbonyldiméthylméthyloxyphényl]prop-2-èn-1-one (Composé 14) selon la méthode générale 3 précédemment décrite.
Purification par chromatographie sur gel de silice (dichlorométhanelméthanol ; 98/2). Rendement : 70%
RMN 1H DMSO δ ppm : 1.38(s, 6H), 2.19(s, 6H), 3.89(s, 3H), 7.12(dd, J = 7.98, J = 1.71 Hz, 1H), 7.23(d, J = 15.56Hz, 1H), 7.29 (d, J = 1.71 Hz, 1H), 7.38(d, J = 15.7 Hz, 1H), 7.41 (s, 2H), 7.48(d, J = 7.98Hz, 1H)
SM(ES-MS): 401.2(M-1)

### Exemple 6 : synthèse de la 1-[4-bromophényl]-3-[3,5-diméthyl-4-carboxydiméthylméthyloxyphényl]prop-2-èn-1-one

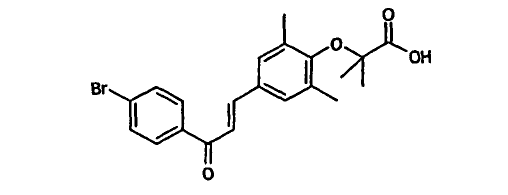

Le procédé permet la préparation de ce composé avec un rendement global de 21%.

### Synthèse du précurseur chimique :

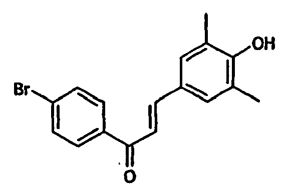

### 1-[4-bromophényl]-3-[3,5-diméthyl-4-hydroxyphényl]prop-2-èn-1-one (Composé 16)

Ce composé a été synthétisé à partir de 4-bromoacétophénone et de 3,5-diméthyl-4-hydroxybénzaldéhyde selon la méthode générale 1 précédemment décrite. Purification par chromatographie sur gel de silice (cyclohexane/acétate d'éthyle : 90/10). Rendement : 37%
RMN 1H DMSO δ ppm : 2,30(s, 6H), 7.32 (s, 2H), 7.56-7.66(m, 3H), 7.75(d, J = 15.27Hz, 1H), 7.90(d, J = 8.70Hz, 2H), 9.82(s, 1H)

### Synthèse de l'ester de tertiobutyle :

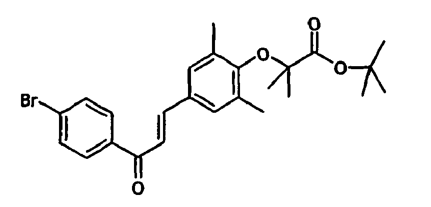

### 1-[4-bromophényl]-3-[3,5-diméthyl-4-tertiobutyloxycarbonyldiméthylméthyloxyphényl]prop-2-èn-1-one (Composé 17)

Ce composé a été synthétisé à partir de 1-[4-bromophényl]-3-[3,5-diméthyl-4-hydroxyphényl]prop-2-èn-1-one (Composé 16) et de bromoisobutyrate de tertiobutyle selon la méthode générale 2 précédemment décrite.
Purification par chromatographie sur gel de silice (cyclohexane/acétate d'éthyle : 90/10). Reniement : 75%

### Acidolyse de l'ester de tertiobutyle :

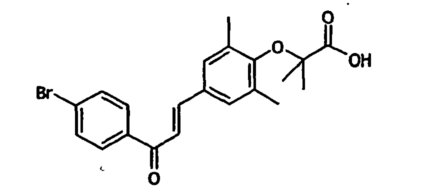

### 1-[4-bromophényl]-3-[3,5-diméthyl-4-carboxydiméthylméthyloxyphényl]prop-2-èn-1-one (Composé 18)

Ce composé a été synthétisé à partir de 1-[4-bromophényl]-3-[3,5-diméthyl-4-tertiobutyloxycarbonyldiméthylméthyloxyphényl]prop-2-èn-1-one (Composé 17) selon la méthode générale 3 précédemment décrite.
Purification par chromatographie sur gel de silice (dichlorométhane/méthanol : 98/2). Rendement : 38 %
RMN 1H DMSO δ ppm : 1.39 (s, 6H), 2.22 (s, 6H), 7.58 (s, 2H), 7.65 (d, J = 15.39Hz, 1H), 7.84-7.77 (m, 3H), 8.09 (d, J = 8.19Hz, 2H), 13.01 (s, 1H)
SM(ES-MS) : 417.2(M-1)

## Revendications

1. Procédé de préparation de dérivés de 1,3-diphénylprop-2-èn-1-one substitués par un groupement carboxyalkyloxy ou carboxyalkylthio, comprenant les étapes suivantes :
(i) une mise en contact d'au moins un dérivé de 1,3-diphénylprop-2-èn-1-one substitué sur un des deux groupes phényle par un groupement hydroxyle ou thiol avec au moins un composé halogéné de formule générale (II) : 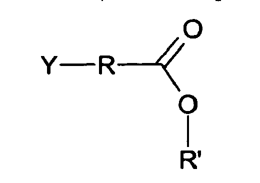 dans laquelle Y représente un atome d'halogène, R est une chaîne alkyle de C1-C24 et R' est un groupement tertiobutyle, choisi comme groupement protecteur acido-sensible de l'acide carboxylique;
(ii) acidolyse de l'ester obtenu à l'étape (i) avec de l'acide trifluoroacétique.

2. Procédé selon la revendication 1, **caractérisé en ce que** le groupement R est une chaîne alkyle de C1-C10 substituée par un ou plusieurs radicaux hydrocarbonés, saturés, linéaires ou cycliques ayant de 1 à 12 atomes de carbone.

3. Procédé selon l'une quelconque des revendications précédentes, **caractérisé en ce que** l'étape (i) est réalisée à une température comprise entre 25 et 120°C et plus préférentiellement entre 80 et 120°C.

4. Procédé selon l'une quelconque des revendications précédentes, **caractérisé en ce que** l'étape (i) est réalisée en présence d'un catalyseur.

5. Procédé selon l'une quelconque des revendications précédentes, **caractérisé en ce que** l'étape (i) est réalisée en présence du carbonate de potassium ou de césium, à titre de catalyseur.

6. Procédé selon l'une quelconque des revendications précédentes, **caractérisé en ce que** l'étape (i) est reproduite en ajoutant plusieurs fois du composé halogéné de formule générale (II) et si nécessaire du catalyseur.

7. Procédé selon l'une quelconque des revendications précédentes, **caractérisé en ce que** le dérivé de 1,3-diphénylprop-2-èn-1-one substitué par un groupement hydroxyle ou thiol utilisé dans le cadre de l'étape (i) est obtenu par une réaction de Claisen Schmidt en milieu acide ou basique d'un composé de type acétophénone avec un dérivé thio- ou hydroxy-benzaldéhyde, ou d'un dérivé de thio- ou hydroxy-acétophénone avec un dérivé de type benzaldéhyde.

8. Procédé selon l'une quelconque des revendications précédentes, **caractérisé en ce que** la quantité d'acide trifluoroacétique utilisée lors de l'étape (ii) est de 1 à 20 équivalents, et préférentiellement de 8 à 12 équivalents.

9. Procédé selon l'une quelconque des revendications précédentes, **caractérisé en ce que** l'étape (ii) est réalisée à une température de 0 à 100°C et plus préférentiellement de 18 à 25°C.

10. Procédé selon l'une des revendications précédentes, **caractérisé en ce que** le produit préparé répond à la formule générale suivante : 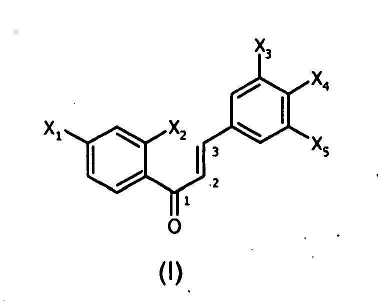 dans laquelle :
X1 représente un halogène ou un groupement -R1 ou un groupement répondant à la formule suivante : -G1-R1 ;
X2 représente un atome d'hydrogène ou un groupement thionitroso ou un groupement alkyloxy ou un groupement alkylcarbonyloxy ou un groupement alkylthio ou un groupement alkylcarbonylthio ;
X3 représente un groupement -R3 ou un groupement répondant à la formule suivante : -G3-R3 ;
X4 représente un halogène ou un groupement thionitroso ou un groupement -R4 ou un groupement répondant à la formule suivante : -G4-R4 ;
X5 représente un groupement -R5 ou un groupement répondant à la formule suivante : -G5-R5 ;
R1, R3, R4, R5, identiques ou différents, représentent un atome d'hydrogène ou un groupement alkyle substitué ou non par une fonction acide carboxylique ;
G1, G3, G4, G5, identiques ou différents, représentent un atome d'oxygène ou de soufre ;
avec un des groupements X1, X3, X4 ou X5 répondant à la formule -G1-R1, -G3-R3, -G4-R4, ou -G5-R5 respectivement, dans laquelle R1, R3, R4 ou R5 respectivement est un radical alkyle portant une fonction acide carboxylique.

## Claims

1. Method of preparation of 1,3-diphenylprop-2-en-1-one derivatives substituted by a carboxyalkyloxy or carboxyalkylthio group, comprising the following steps :
(i) contacting at least one 1,3-diphenylprop-2-en-1-one derivative substituted on one of the two phenyl groups by a hydroxyl or thiol group with at least one halogenated compound represented by general formula (II) : 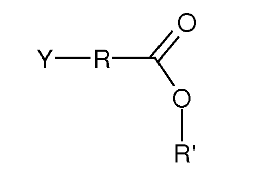 in which Y represents a halogen atom, R is a C1-C24 alkyl chain and R' is a tertiobutyl group, chosen as acid-labile protective group of carboxylic acid;
(ii) acid hydrolysis of the ester obtained in step (i) with trifluoroacetic acid.

2. Method according to claim 1, **characterized in that** the R group is a C1-C10 alkyl chain, substituted by one or more hydrocarbon groups, saturated, linear or cyclic containing from 1 to 12 carbon atoms.

3. Method according to any one of the previous claims, **characterized in that** step (i) is carried out at a temperature comprised between 25 and 120°C and more preferably between 80 and 120 °C.

4. Method according to any one of the previous claims, **characterized in that** step (i) is carried out in the presence of a catalyst.

5. Method according to any one of the previous claims, **characterized in that** step (i) is carried out in the presence of cesium or potassium carbonate as catalyst.

6. Method according to any one of the previous claims, **characterized in that** step (i) is repeated by several additions of the halogenated compound represented by general formula (II) and if necessary of the catalyst.

7. Method according to any one of the previous claims, **characterized in that** the 1,3-diphenylprop-2-en-1-one derivative substituted by a hydroxyl or thiol group, which is used in step (i) is obtained by a Claisen-Schmidt reaction in acidic or basic medium of a compound of the type acetophenone with a thio- or hydroxy-benzaldehyde derivative, or of a thio- or hydroxy-acetophenone derivative with a compound of the benzaldehyde type.

8. Method according to any one of the previous claims, **characterized in that** the amount of trifluoroacetic acid which is used in step (ii) is from 1 to 20 equivalents, and preferably from 8 to 12 equivalents.

9. Method according to any one of the previous claims, **characterized in that** step (ii) is carried out at a temperature of 0 to 100°C and more preferably 18 to 25°C.

10. Method according to any one of the previous claims, **characterized in that** the product so prepared is represented by the following general formula : 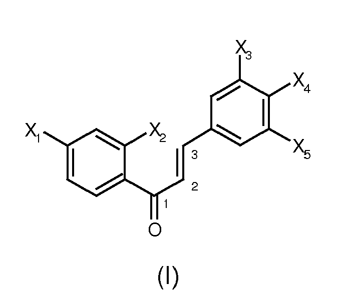 in which :
X1 represents a halogen or a -R1 group or a group corresponding to the following formula : -G1-R1;
X2 represents a hydrogen atom or a thionitroso group or an alkyloxy group or an alkylcarbonyloxy group or an alkylthio group or an alkylcarbonylthio group;
X3 represents a -R3 group or a group corresponding to the following formula : -G3-R3;
X4 represents a halogen or a thionitroso group or a -R4 group or a group corresponding to the following formula : -G4-R4;
X5 represents a -R5 group or a group corresponding to the following formula : -G5-R5;
R1, R3, R4, R5, which are the same or different, represent a hydrogen atom or an alkyl group substituted or not by a carboxylic acid function;
G1, G3, G4, G5, which are the same or different, represent an oxygen or sulfur atom;
with one of the groups X1, X3, X4 or X5 corresponding to the formula -G1-R1, -G3-R3, -G4-R4, or -G5-R5 respectively, in which R1, R3, R4 or R5 respectively is an alkyl group containing a carboxylic acid function.

## Patentansprüche

1. Verfahren zur Herstellung von 1,3-Diphenylprop-2-en-1-on-Derivaten, die mit einer Carboxyalkyloxy- oder Carboxyalkylthio-Gruppe substituiert sind, umfassend die folgenden Schritte:
(i) Inkontaktbringen wenigstens eines 1,3-Diphenylprop-2-en-1-on-Derivats, das an einer der beiden Phenylgruppen mit einer Hydroxy- oder Thiolgruppe substituiert ist, mit wenigstens einer halogenierten Verbindung der allgemeinen Formel (II): 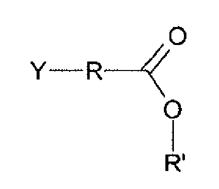 in der Y für ein Halogenatom steht, R eine C1-C24-Alkylkette ist und R' eine tertiäre Butylgruppe ist, die als eine für Carbonsäuren säureempfindliche Schutzgruppe ausgewählt ist;
(ii) saure Hydrolyse des in Schritt (i) erhaltenen Esters mit Trifluoressigsäure.

2. Verfahren gemäß Anspruch 1, **dadurch gekennzeichnet, dass** die Gruppe R eine C1-C10-Alkylkette ist, die mit einem oder mehreren gesättigten, linearen oder cyclischen Kohlenwasserstoffresten mit 1 bis 12 Kohlenstoffatomen substituiert ist.

3. Verfahren gemäß einem der vorhergehenden Ansprüche, **dadurch gekennzeichnet, dass** der Schritt (i) bei einer Temperatur zwischen 25 und 120°C und bevorzugter zwischen 80 und 120°C durchgeführt wird.

4. Verfahren gemäß einem der vorhergehenden Ansprüche, **dadurch gekennzeichnet, dass** der Schritt (i) in Gegenwart eines Katalysators durchgeführt wird.

5. Verfahren gemäß einem der vorhergehenden Ansprüche, **dadurch gekennzeichnet, dass** der Schritt (i) in Gegenwart von Kalium- oder Cäsiumcarbonat als Katalysator durchgeführt wird.

6. Verfahren gemäß einem der vorhergehenden Ansprüche, **dadurch gekennzeichnet, dass** der Schritt (i) unter mehrfacher Zugabe der halogenierten Verbindung der allgemeinen Formel (II) und, falls erforderlich, des Katalysators wiederholt wird.

7. Verfahren gemäß einem der vorhergehenden Ansprüche, **dadurch gekennzeichnet, dass** das im Rahmen des Schrittes (i) eingesetzte Derivat von 1,3-Diphenylprop-2-en-1-on, das mit einer Hydroxy- oder Thiolgruppe substituiert ist, durch eine Claisen-Schmidt-Reaktion einer Verbindung vom Acetophenon-Typ mit einem Thio- oder Hydroxybenzaldehyd-Derivat oder eines Thio- oder Hydroxyacetophenon-Derivats mit einem Derivat vom Benzaldehyd-Typ in saurem oder basischem Milieu erhalten wird.

8. Verfahren gemäß einem der vorhergehenden Ansprüche, **dadurch gekennzeichnet, dass** die in Schritt (ii) eingesetzte Menge an Trifluoressigsäure 1 bis 20 Äquivalente und bevorzugt 8 bis 12 Äquivalente beträgt.

9. Verfahren gemäß einem der vorhergehenden Ansprüche, **dadurch gekennzeichnet, dass** der Schritt (ii) bei einer Temperatur von 0 bis 100°C und bevorzugter von 18 bis 25°C durchgeführt wird.

10. Verfahren gemäß einem der vorhergehenden Ansprüche, **dadurch gekennzeichnet, dass** das dargestellte Produkt der folgenden allgemeinen Formel entspricht: 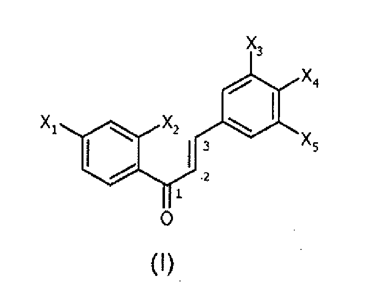 in der:
X1 für ein Halogen oder eine Gruppe -R1 oder eine Gruppe steht, die der folgenden Formel entspricht: -G1-R1;
X2 für ein Wasserstoffatom oder eine Thionitroso-Gruppe oder eine Alkyloxy-Gruppe oder eine Alkylcarbonyloxy-Gruppe oder eine Alkylthio-Gruppe oder eine Alkylcarbonylthio-Gruppe steht;
X3 für eine Gruppe -R3 oder eine Gruppe steht, die der folgenden Formel entspricht: -G3-R3;
X4 für ein Halogen oder eine Thionitroso-Gruppe oder eine Gruppe -R4 oder eine Gruppe steht, die der folgenden Formel entspricht: -G4-R4;
X5 für eine Gruppe -R5 oder eine Gruppe steht, die der folgenden Formel entspricht: -G5-R5;
R1, R3, R4, R5, die identisch oder verschieden sind, für ein Wasserstoffatom oder eine Alkylgruppe stehen, die nicht substituiert ist oder mit einer Carbonsäure-Funktion substituiert ist;
G1, G3, G4, G5, die identisch oder verschieden sind, für ein Sauerstoff- oder Schwefelatom stehen;
wobei eine Gruppe der Gruppen X1, X3, X4 oder X5 der Formel -G1-R1, -G3-R3, -G4-R4 oder beziehungsweise -G5-R5 entspricht, in der R1, R3, R4 oder beziehungsweise R5 ein Alkylrest ist, der eine Carbonsäure-Funktion trägt.
